# EUROPEAN PATENT APPLICATION

(11) **EP 2 352 027 A1**
(43) Date of publication of application: **03.08.2011**
(21) Application number: 09821863.9
(22) Date of filing: 16.07.2009
(51) Int. Cl.: G01N 33/543, G01N 33/53

(54) **METHOD AND REAGENT KIT FOR IMMUNOLOGICAL MEASUREMENT**

(30) Priority: 22.10.2008 JP 2008272270
(71) Applicant: Alfresa Pharma Corporation, Chuo-ku Osaka-shi Osaka 540-8575 (JP)
(72) Inventor: TANAKA, Mutsumi, Ibaraki-shi Osaka 567-0806 (JP)
(74) Representative: Mathys & Squire LLP
(86) International application number: PCT/JP2009/062865
(87) International publication number: WO 2010/047163

(57) **Abstract**

The invention provides a method of the invention for measuring an analyte in a sample, which comprises (a) mixing a sample containing the analyte, a complex comprising a plurality of molecules of a substance that specifically binds to the analyte, and microparticles each having the analyte or an analog of the analyte bound to an insoluble support; and (b) measuring an agglutination reaction of the microparticles in a mixture obtained in the step (a). In the method of the invention, for example, an antibody is used in the form of a complex of a plurality of molecules of the antibody, rather than in the form of a single molecule of the antibody. Therefore, the sensitivity to the measurement is increased, and the measurement in a lower concentration range becomes possible.

## Description

### Technical Field

The present invention relates to a method for immunological measurement using substance-bound microparticles. In particular, the present invention relates to a method and a reagent kit for immunologically measuring a trace component using an antigen-antibody reaction, primarily in the fields of industry, environment, and clinical research.

### Background Art

Recently, various researches such as clinical researches have become automated and reduced in measurement time. In the researches, methods of measurement using an immune reaction are widely used for measurement of a substance present in a biological sample. Examples of methods for immunological measurement include various methods such as radioimmunoassay (RIA), enzyme immunoassay (EIA), turbidimetric immunoassay, latex agglutination, colloidal gold agglutination, and immunochromatography. Among such methods, latex agglutination or colloidal gold agglutination allows for measurement in a homogeneous system not requiring separation or washing of the reaction solution, and are thus suitable for automated measurement and short-time measurement. In particular, since colloidal gold particles have a size of 5 to 100 nm, which is smaller than the size of latex particles, colloidal gold particles can be more conveniently used in the measurement for trace substances (Patent Documents 1 and 2).

The main reactive component in those measurement methods is microparticles, such as latex particles or colloidal gold particles, each having a substance that specifically reacts (for example, binds) with an analyte bound thereto. Where a substance that specifically reacts with an analyte, which has multiple sites for specifically reacting with the analyte, is bound to microparticles, the reaction of an analyte with the substance that specifically reacts with the analyte bound to each of microparticles results in the agglutination of the microparticles, and the concentration of the analyte is calculated from the extent of the agglutination.

However, where a substance that specifically reacts with an analyte, which has only one site for specifically reacting with the analyte, is bound to microparticles, when an analyte is reacted with the substance, the reaction (for example, binding) of the substance with the analyte results in only at the one site of the substance, which fails to genetrate the agglutination of microparticles. In the case, the analyte is measured with a competitive assay, by placing also a competitor having multiple sites for reacting with the analyte concomitantly in the measurement system to effect the agglutination of microparticles and measuring the extent of agglutination depending on the concentration of the analyte.

Alternatively, disclosed is a method for measuring an analyte, by placing, in a reaction system for an agglutination due to the reaction of microparticles such as latex particles or colloidal gold particles each having a plurality of molecules of an analyte or an analog of the analyte bound thereto with a substance that specifically binds to the analyte, an analyte concomitantly so as to inhibit the reaction for the agglutination, and measuring the extent of inhibition (Non-Patent Document 1). In this method, in the case of using an antibody in the form of a single molecule as the substance that specifically binds to the analyte, when the antibody is bound to the analyte or the latex particles or colloidal particles each having a plurality of hapten molecules bound thereto, insufficient agglutination may be effected, failing to obtain the measurement sensitivity suitable for practical use.

### Prior Art Documents

### Patent Documents

Patent Document 1: Japanese Laid-Open Patent Publication No. 2005-283250
Patent Document 2: Japanese Laid-Open Patent Publication No. 2004-325192

### Non-Patent Documents

Non-Patent Document 1: Mitsuhiro Yamada, The Journal of Clinical Laboratory Instruments and Reagents, Vol. 19, No. 4, pp. 523-528, 1996

### Summary of Invention

### Problems to be Solved by the Invention

In regard to a method for measuring an analyte in a conventional reaction system using microparticles, i.e., a method for measuring an analyte, by placing the analyte concomitantly in a reaction system for an agglutination due to the reaction of latex particles or colloidal gold particles each having a plurality of molecules of the analyte or an analog of the analyte bound thereto with a substance that specifically binds to the analyte so as to inhibit the reaction for the agglutination, and measuring the extent of inhibition, it is an object of the invention to provide a method for measurement which allows for measurement of analyte with an increased measurement sensitivity and in a broader concentration range, and a kit therefor.

### Means for Solving the Problems

The present invention has been accomplished through finding that, in the foregoing measurement method, the agglutination reaction is dramatically enhanced using a substance that specifically binds to an analyte in the form of a complex having a plurality of molecules of the specific binding substance bound therein, rather than in the form of a single molecule, with microparticles each having a plurality of molecules of the analyte or an analog of the analyte bound thereto, and this results in significantly increased sensitivity to the measurement of the analyte. That is, the measurement method of the invention is based on the phenomenon in which when an analyte present in a sample, such a complex, and microparticles each having a plurality of molecules of the analyte or an analog of the analyte bound thereto are concomitantly placed in a reaction solution for the agglutination reaction, the agglutination reaction is inhibited in a manner dependent on the concentration of the analyte which is derived from the sample.

The present invention provides a method for measuring an analyte in a sample, the method comprising:
(a) mixing:
   a sample containing the analyte,
   a complex comprising a plurality of molecules of a substance that specifically binds to the analyte, and
   microparticles each having the analyte or an analog of the analyte bound to an insoluble support; and
(b) measuring an agglutination reaction of the microparticles in a mixture obtained in the step (a).

The present invention also provides a reagent kit for measurement comprising:
a first reagent, which comprises a complex comprising a plurality of molecules of a substance that specifically binds to an analyte; and
a second reagent, which comprises microparticles each having an analyte or an analog of the analyte bound to an insoluble support.

In an embodiment, the complex is a substance having a plurality of molecules of an antibody against the analyte bound therein.

In an embodiment, the analyte is a hapten and the analog of the analyte is a hapten-bound protein.

In another embodiment, the analog of the analyte is (i) a substance having a site of the analyte, (ii) a structural analog of the substance having the site, or (iii) a substance having a plurality of molecules of the substance having the site or the structural analog bound therein, which can be recognized by and bound to the substance that specifically binds to the analyte.

In a further embodiment, the insoluble support is latex or colloidal gold.

### Effects of Invention

According to the present invention, the agglutination reaction is dramatically enhanced using a substance that specifically binds to an analyte in the form of a complex having a plurality of molecules of the substance bound therein, rather than in the form of a single molecule, with microparticles (such as latex particles and colloidal gold particles) each having a plurality of molecules of the analyte or an analog of the analyte bound thereto, and this results in significantly increased sensitivity to the measurement of the analyte. Accordingly, the measurement in such a low concentration range that could not be made with any conventional method becomes possible, and thus the analyte can be measured in a broader concentration range.

Meanwhile, in conventional methods, when the progression of agglutination reaction does not occur, an agglutination enhancer (such as polyethylene glycol and chondroitin sulfate) is added to the reaction solution, thereby leading to increase in viscosity of the reaction solution, thus making it difficult to handle the reaction solution and affecting reproducibility, which are problematic. In contrast, according to the method of the invention, the agglutination is sufficiently effected without any agglutination enhancer, and thus the method of the invention is favorable in handling and reproducibility

### Brief Description of Drawings

[Fig. 1] Fig. 1 is a graph showing the relationship between the concentration of diacetylspermine and the amount of change in absorbance in diacetylspermine measurement 1 with first reagents (a), (b), and (c) (Example 7).
[Fig. 2] Fig. 2 is a graph showing the relationship between the concentration of diacetylspermine and the amount of change in absorbance in diacetylspermine measurement 2 with first reagents (d) and (e) (Example 10).

### Mode for Carrying Out the Invention

### (Sample)

In the present invention, examples of analyte-containing samples to be subjected to the measurement include biological samples such as blood, blood plasma, serum, urine, feces (suspension), cerebrospinal fluid, and ascites fluid; and samples obtained from the environment or extracts thereof; and the like.

### (Analyte)

The analyte is not particularly limited as long as a substance that specifically binds to the analyte can exist or the substance can be produced. Examples of the analyte include proteins such as albumin, hemoglobin, hemoglobin A1c, myoglobin, transferrin, lactoferrin, cystatin C, ferritin, α-fetoprotein, carcinoembryonic antigen, CA19-9, prostate-specific antigen, C-reactive protein (CRP), fibrin degradation product (FDP), pepsinogens I and II, and collagen; lipoproteins such as high-density lipoprotein, low-density lipoprotein, and very low-density lipoprotein; nucleic acids such as deoxyribonucleic acid and ribonucleic acid; enzymes such as alkaline phosphatase, lactate dehydrogenase, lipase, and amylase; immunoglobulins such as IgG, IgM, IgA, IgD, and IgE; antigens and antibodies associated with infectious diseases, such as hepatitis B virus, hepatitis C virus, human immunodeficiency virus, and *Helicobacter pylori* and antibodies thereto; polyamines such as spermine, spermidine, putrescine, and diacetylspermine, and physiologically active substances; drugs such as haloperidol and bromperidol; and hormones such as sex hormone.

### (Complex of substance that specifically binds to analyte)

An example of the substance that specifically binds to an analyte (hereinafter sometimes referred to as a "specific binding substance") is an antibody or an antigen that can be used in an immunological measurement using an immune reaction. For example, substances having binding affinity, such as antibodies or antigens, receptors, lectin, deoxyribonucleic acid (DNA), and ribonucleic acid (RNA), can be used. Preferably, a polyclonal antibody or a monoclonal antibody is used because they can specifically recognize an analyte and are easily recognized by an analyte or its analog bound to an insoluble support that will be described in detail below.

The complex of a specific binding substance (hereinafter sometimes simply referred to as a "complex") is not particularly limited as long as it contains a plurality of single molecules of the specific binding substance. Examples of complexes include those produced by chemical binding of a plurality of single molecules of a specific binding substances to each other (for example, with a linker); those produced by binding of a plurality of molecules of a specific binding substance via a carrier capable of specifically binding a plurality of molecules of the specific binding substance; and those produced by applying a tag such as biotin to a specific binding substance by chemical modification and forming a complex from a plurality of molecules of the tagged specific binding substance via a substance that specifically binds to the tag. Here, the term "plurality" refers to "more than one", so it means that there are 2, 3, 4, 5, 6, or more of single molecules of the specific binding substance, depending on the mode of conjugation of the complex.

Binding of single molecules of a specific binding substance to each other is attained by chemical linkage of an analyte or a portion thereof to a support directly or via a linker using a functional group such as an amino group, a carboxyl group or a thiol group present in the analyte or a portion thereof, and various ones are known, depending on the structure of the analyte or a portion thereof (Seikagaku Jikken Hou (Biochemical Experiment Methods) 11, Enzyme Immunoassay, authored by P. Tijssen, edited by Eiji Ishikawa, p. 252, 1989, Tokyo Kagaku Dozin Co., Ltd.). Examples of reagents for forming a chemical linkage include acylating agents and alkylating agents. N-hydroxysuccinimido esters, which are obtained by activating a carboxyl group, and maleimides, which are used under weakly alkaline conditions, are preferable.

A complex may be formed using biotinylation of a specific binding substance and binding of the biotinylated specific binding substance to a substance (such as avidin, streptoavidin, neutravidin) that specifically binds to biotin.

Examples of carriers capable of specifically binding a plurality of molecules of a specific binding substance include antibodies that specifically bind to the specific binding substance; substances having a plurality of molecules of the antibody that specifically binds to the specific binding substance chemically bound with an anchor or the like; substances formed via specific binding of the biotinylated antibody that specifically binds to the specific binding substance with avidin, streptoavidin, neutravidin, or the like; and like carriers.

### (Analog of analyte)

The analog of the analyte is not particularly limited as long as it specifically binds to a specific binding substance. The analog of the analyte is (i) a substance having a site of the analyte, (ii) a structural analog of the substance having the site, or (iii) a substance having a plurality of molecules of the substance having the site or the structural analog bound therein, which can be recognized by and bound to the substance that specifically binds to the analyte. In the case that the analyte is a hapten, the analog of the analyte is, for example, a hapten-bound protein.

A plurality of molecules of an analyte or its analog may be bound to a support which is different from an insoluble support that will be described in detail below. Such a support is suitably selected from albumin, hemocyanin, thyroglobulin, fibrinogen, enzyme, and the like derived from various animals. In the present invention, bovine serum albumin (BSA) is preferable. It is preferable that about 4 to 40 molecules of an analytes or an analog thereof are bound per molecule of a support. Binding of the analyte or the analog and such a support can be created according to a method commonly used by a person skilled in the art. Such a method may be the same as the method used for binding the foregoing single specific binding substance molecules to each other. The analog of the analyte may be bound to an insoluble support.

### (Microparticles)

In the present invention, any microparticles that can be used for an immunoassay reagent can be used as an insoluble support for binding an analyte or its analog. Latex and metal colloid are preferably used. In the case of metal colloid, gold colloid is preferable in view of generally ease to use. Commercially available colloidal gold particles may be used, or colloidal gold particles may be prepared by a method commonly used by those skilled in the art (e.g., a method of reducing chloroauric acid with sodium citrate). The particle size of the colloidal gold particles is usually in the range of 10 nm to 100 nm, preferably in the range of 30 nm to 60 nm.

Microparticles each having the specific binding substance as mentioned above bound thereto (hereinafter sometimes referred to as "bound microparticles") for use in the method of the present invention may be prepared, for example, using microparticles of colloidal gold particles, in the following manner. First, usually 0.1 mg to 100 mg, preferably 1 mg to 10 mg, of an analyte or analog of the analyte is added to 1 L of a colloidal solution containing gold particles (having an absorbance at 540 nm of about 2.0), and the mixture is stirred under refrigeration or at room temperature for 5 minutes to 24 hours. Then, the mixture is subjected to blocking with bovine serum albumin (BSA) or the like and centrifugation, and thus the desired bound microparticles (bound colloidal gold particles, in this case) can be obtained. The obtained microparticles are dispersed in a buffer to attain a concentration required for measurement. The pH of the buffer is preferably 5 to 9, and the concentration thereof is preferably 1 to 100 mM. For example, phosphate buffer, Tris-HCl buffer, succinate buffer, or Good's buffers such as glycylglycine, MES (2-(N-morpholino)ethanesulfonic acid), HEPES (2-[4-(2-Hydroxyethyl)-1-piperadinyl] ethanesulfonic acid), TES (N-tris (hydroxymethyl)methyl-2-aminoethanesulfonic acid), MOPS (3-(N-morpholino)propanesulfonic acid), PIPES (piperazine-1,4-bis(2- ethanesulfonic acid)), and Bis-Tris (bis(2-hydroxyethyl)iminotris (hydroxymethyl)methane) can be preferably used as the buffer.

The buffer may contain sugars and sugar alcohols, sodium azide, albumin, salts such as sodium chloride, and additives such as preservatives, as necessary. Examples of the sugars and sugar alcohols include glucose, mannose, saccharose, lactose, maltose, mannitol, and sorbitol, and the concentration thereof is preferably 0.01 to 10 w/v%. As for the albumin, bovine serum albumin (BSA) is preferably used, and the concentration thereof is preferably 0.001 to 1 w/v%. As for the preservatives, sodium azide is preferably used, and the concentration thereof is preferably 0.01 to 0.5 w/v%. Examples of other additives include Tween 20, polyethylene glycol lauryl ether, 5-bromosalicylic acid, sodium salicylate, sodium benzoate, sodium benzenesulfonate, phenol, and thymol.

### (Method for measuring analyte)

According to the invention, the method for measuring an analyte in a sample includes the steps of
(a) mixing:
   a sample containing the analyte,
   a complex including a plurality of molecules of a substance that specifically binds to the analyte (i.e., a specific binding substance), and
   microparticles having the analyte or an analog of the analyte bound to an insoluble support (hereinafter sometimes referred to as "bound microparticles"); and
(b) measuring an agglutination reaction of the microparticles in a mixture obtained in the step (a).

In this method, when an analyte is present concomitantly in the reaction solution containing such a complex and bound microparticles, the agglutination reaction due to the reaction (for example, binding) of a specific binding substance in the complex with an analyte or an analog thereof located on the bound microparticles is inhibited in a manner dependent on the concentration of the analyte, to be contained in the sample. Therefore, the extent of inhibition is measured mechanically

For the binding reaction of the analyte with the complex and for the agglutination reaction of the bound microparticles, reaction conditions such as reaction temperature, pH, the kind of buffer, the kind and concentration of concomitant salt, and other concomitant substances are the same as the conditions selected for conventional immunological reactions and can be suitably selected by a person skilled in the art. For example, as is commonly performed, water-soluble polymers such as polyethylene glycol, polyvinyl alcohol, dextran, and sodium chondroitin sulfate may be introduced into the reaction system for reaction enhancement.

The method of the present invention is performed, for example, as follows. For a first reaction, a sample which may contain an analyte or a dilution of the sample suitably diluted with a buffer or the like is mixed with a complex having a plurality of molecules of a specific binding substance bound therein. Next, for a second reaction, to this mixture, bound microparticles are added and mixed. Any specific binding substance in the complex which has not bound to the analyte or an analog thereof in the first reaction binds to the analyte or an analog thereof located on the bound microparticles in the second reaction, which results in the agglutination reaction of the bound microparticles via the complex. This agglutination reaction is dependent on the amount of the complex which binds to the bound microparticles, and it therefore depends on the amount of the complex which has not bound to the analyte in the first reaction. That is, depending on the amount of the analyte in the sample of the first reaction, the agglutination reaction of the second reaction is decreased.

Alternatively, the measurement method of the present invention may be performed as follows: a sample which may contain an analyte or a dilution of the sample suitably diluted with a buffer or the like is first mixed with bound microparticles, and a complex having a plurality of molecules of a specific binding substance bound therein is then added to this mixture and they are mixed. The agglutination reaction may be effected on the addition of the complex, but is competitively inhibited with an analyte to be from the sample concomitantly in the reaction solution. Therefore, the concentration of the analyte can be determined by measuring the change in absorbance due to the agglutination reaction.

Using microparticles of gold colloid, the change in absorbance due to agglutination reaction at a predetermined wavelength is measured. The amount of the analyte in the sample can be easily determined by applying the results of the measurement to a calibration curve created beforehand. The calibration curve represents the relationship between the change in absorbance due to colloidal gold agglutination reaction and the amount of the analyte. It should be noted that a qualitative analysis and a semi-quantitative analysis can also be performed, in which the sample is determined as negative when the change in absorbance is less than a certain value and as positive when the change in absorbance is not less than the certain value.

Either a single wavelength measurement or a dual wavelength measurement may be used with gold colloid to measure the change in absorbance after the start of the reaction. When the dual wavelength measurement is used, the change in absorbance is measured at the first wavelength of 610 nm to 800 nm, preferably 630 nm to 750 nm, and the second wavelength of 360 nm to 580 nm, preferably 500 nm to 550 nm. When the single wavelength measurement is used, the change in absorbance can be measured at a wavelength in the wavelength region of either one of the first wavelength or the second wavelength used in the above-described dual wavelength measurement. In the method of the present invention, the change in absorbance refers to values obtained by the two measurement methods described below, and either of the values can be used:
(1) the absorbance of the reaction mixture is measured twice at an appropriate interval after the start of the reaction, and the difference between the two measured values is used as the change in absorbance; or
(2) the absorbance of the reaction mixture is continuously measured after the start of the reaction, and the rate of change in the absorbance per unit time (in some cases, the maximum rate of change) is used as the change in absorbance.

For the above-described measurement, spectrophotometers, microplate readers, biochemical automatic analyzer, and the like can be used. In particular, the method of the present invention can be applied to the measurement with a biochemical automatic analyzer to measure a number of samples in a short period of time.

### (Reagent kit for measurement)

According to the present invention, a reagent kit for measurement for use in the method of the present invention is provided. This kit contains a first reagent which contains a complex including a plurality of molecules of a substance that can specifically bind to an analyte; and a second reagent which contains microparticles each having an analyte or an analog of the analyte bound to an insoluble support.

The above described reagents may be provided in any form, and preferably are provided in a state where the reagents are individually sealed and packaged. The above-described kit may contain a reference standard of the analyte for use in creation of a calibration curve, a buffer for preparing a solution by dissolving each material at an appropriate concentration at the time of use, instructions for use, and the like.

### Examples

Hereinafter, the present invention will be described even more specifically by way of examples. However, the present invention is not limited by the examples.

### (Example 1: Preparation of gold colloidal solution)

First, 2 mL of a 10 w/v% chloroauric acid solution was added to 1 L of distilled water at 95°C under stirring, and after one minute, 10 mL of a 2 w/v% sodium citrate solution was added thereto. The resulting mixture was stirred for further 20 minutes and then cooled to 30°C. After cooling, the pH was adjusted to 7.1 with 0.1 w/v% potassium carbonate.

### (Example 2: Preparation of diacetylspermine-bound colloidal gold reagent)

Diacetylspermine-bound BSA (Trans Genic Inc.) was diluted with 10 mM HEPES (pH 7.1) containing 0.25 w/v% of sodium azide to a concentration of 5 mg/mL. 100 mL of this solution was added to about 1 L of the colloidal gold solution as prepared in Example 1 above, and the mixture was stirred at room temperature for 2 hours. Moreover, 110 mL of 10 mM HEPES (pH 7.1) containing 5.46 w/v% of mannitol, 0.5 w/v% of BSA, and 0.05 w/v% of sodium azide was added, stirring was performed at room temperature for 1 hour, and centrifugation was performed at 8000 rpm for 40 minutes to remove a supernatant. To the resultant residue was added about 1 L of 5 mM HEPES (pH 7.5) containing 3 w/v% of mannitol, 0.1 w/v% of BSA, and 0.05 w/v% of sodium azide (solution A) to obtain a dispersion of colloidal gold particles, and thereafter, the dispersion was centrifuged at 8000 rpm for 40 minutes to remove a supernatant. To the resultant residue was added solution A to obtain a dispersion of colloidal gold particles to the total amount of 70 mL, giving a diacetylspermine-bound colloidal gold solution.

Then, 280 mL of solution A was added to 70 mL of the diacetylspermine-bound colloidal gold solution, giving a diacetylspermine-bound colloidal gold reagent.

### (Example 3: Preparation of complex of anti-diacetylspermine antibody and anti-mouse IgG antibody)

An anti-diacetylspermine antibody (Trans Genic Inc.) and an anti-mouse IgG antibody (Production of Antibodies, Reagents for Immunology and Services) were mixed at a molar concentration ratio of the anti-diacetylspermine antibody to the anti-mouse IgG of 2:1, giving a complex of anti-diacetylspermine antibody and anti-mouse IgG antibody.

### (Example 4: Preparation of anti-mouse IgG antibody-bound avidin)

An anti-mouse IgG antibody was biotinated using Biotin Labeling Kit-SH (Dojindo Molecular Technologies, Inc.). The biotinated IgG antibody and avidin were mixed at a molar concentration ratio of the biotinated IgG antibody to the avidin of 4:1, giving anti-mouse IgG antibody-bound avidin.

### (Example 5: Preparation of complex of anti-diacetylspermine antibody and anti-mouse IgG antibody-bound avidin)

The anti-mouse IgG antibody-bound avidin as prepared in Example 3 and an anti-diacetylspermine antibody were mixed at a molar concentration ratio of the anti-mouse IgG antibody-bound avidin to the anti-diacetylspermine antibody of 1:4, giving a complex of anti-diacetylspermine antibody and anti-mouse IgG antibody-bound avidin.

### (Example 6: Preparation of first reagent for diacetylspermine measurement 1)

The complex of anti-diacetylspermine antibody and anti-mouse IgG antibody as prepared in Example 3 was added to a 0.2 M PIPES (pH 6.5) solution containing 1.0 w/v% of sodium chloride, 0.5 w/v% of EDTA, 2.5 w/v% polyethylene glycol, and 0.35 w/v% of polyoxyethylene lauryl ether (solution B) so as to attain 4.94×10⁻¹² mol/mL (amount of anti-diacetylspermine antibody: 1.48 µg/mL reagent), giving a first reagent (a) for diacetylspermine measurement 1.

Separately, the complex of anti-diacetylspermine antibody and anti-mouse IgG antibody-bound avidin as prepared in Example 5 above was added to solution B so as to attain 2.47×10⁻¹² mol/mL (amount of anti-diacetylspermine antibody: 1.48 µg/mL reagent), giving a first reagent (b) for diacetylspermine measurement 1.

As a control, an anti-diacetylspermine antibody was added in the form of a single molecule to solution B so as to attain the same concentration (1.48 µg/mL reagent) as those of the foregoing first reagents (a) and (b), giving a first reagent (c) for diacetylspermine measurement 1.

### (Example 7: Diacetylspermine measurement 1)

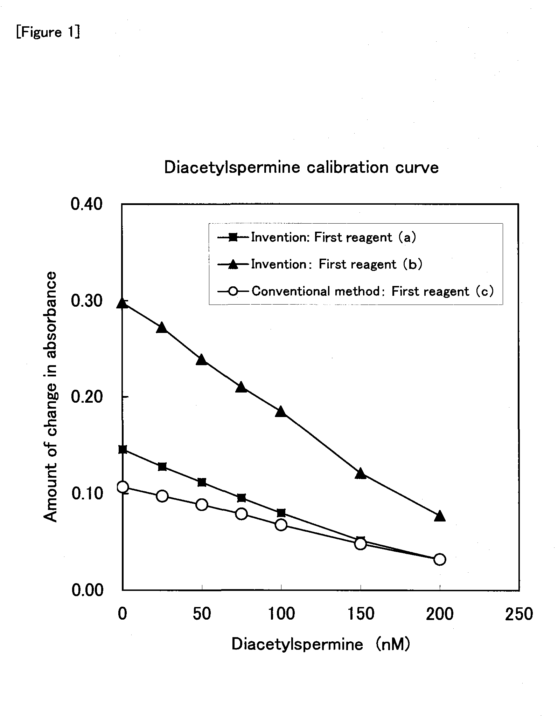

In this example, the three types of first reagents (a), (b), and (c) for diacetylspermine measurement 1 as prepared in Example 6 above were used as the first reagent, and the diacetylspermine-bound colloidal gold reagent as prepared in Example 2 above was used as the second reagent. Diacetylspermine was dissolved in Bis-Tris (pH 7.4) containing 3.0 w/v% of BSA and 1.0 w/v% of sodium chloride so as to attain 0, 25, 50, 75, 100, 150, and 200 nM, giving diacetylspermine-containing samples. Then, 160 µL of the first reagent was added to 10 µL each of the diacetylspermine-containing samples and they were heated at 37°C for about 5 minutes, and 80 µL of the second reagent was added and they were at 37°C for reaction. The amount of change in absorbance was measured with Hitachi 7070 automatic analyzer at photometric points from 18 to 31 at wavelengths of 546 nm and 660 nm. Figure 1 and Table 1 show the relationship between the diacetylspermine concentration and the amount of change in absorbance.

**[Table 1]**

| Diacetylspermine n(M) | First reagent | | |
|---|---|---|---|
| | (a) | (b) | (c) |
| 0 | 0.1455 | 0.2980 | 0.1068 |
| 25 | 0.1279 | 0.2724 | 0.0975 |
| 50 | 0.1118 | 0.2392 | 0.0887 |
| 75 | 0.0954 | 0.2102 | 0.0788 |
| 100 | 0.0798 | 0.1849 | 0.0674 |
| 150 | 0.0515 | 0.1213 | 0.0480 |
| 200 | 0.0320 | 0.0774 | 0.0320 |

As shown in Figure 1 and Table 1, the amount of change in absorbance due to an agglutination reaction was changed in a manner dependent on the concentration of the analyte diacetylspermine. That is, the higher the concentration of diacetylspermine, the lower the amount of change in absorbance. It can be understood that, although these respective first reagents contain the antibody in a same amount, the amount of absorbance change is larger and measurement with higher sensitivity can be obtained to a lower concentration range of diacetylspermine with the first reagents (a) and (b), which contained the antibody in the form of a complex, than with the first reagent (c), which contained the antibody conventionally in the form of a single molecule. Therefore, it was found that, in the measurement of the amount of analyte diacetylspermine in a sample in reference to the amount of change in absorbance in an agglutination reaction, a complex having a plurality of molecules of the antibody bound therein allows for highly sensitive measurement also in a low concentration range, thereby enabling measurement in a broader concentration range.

### (Example 8: Preparation of complex of anti-diacetylspermine antibody and avidin)

An anti-diacetylspermine antibody was biotinylated using Biotin Labeling Kit-SH. The biotinylated anti-diacetylspermine antibody and avidin were mixed at a molar concentration ratio of the biotinylated anti-diacetylspermine antibody to the avidin of 4:1, giving anti-mouse IgG antibody-bound avidin.

### (Example 9: Preparation of first reagent for diacetylspermine measurement 2)

The complex of anti-diacetylspermine antibody and avidin as prepared in Example 8 was added to a 0.2 M Bis-Tris (pH 6.5) solution containing 1.0 w/v% of sodium chloride, 0.2 w/v% of BSA, 2.5 w/v% of polyethylene glycol, and 0.35 w/v% of polyoxyethylene lauryl ether (solution C) so as to attain 2.96×10⁻¹² mol/mL (amount of anti-diacetylspermine antibody: 0.888 µg/mL reagent), giving a first reagent (d) for diacetylspermine measurement 2.

As a control, an anti-diacetylspermine antibody was added to solution C so as to attain the same concentration (0.888 µg/mL reagent) as that of the foregoing first reagent (d), giving a first reagent (e) for diacetylspermine measurement 2.

### (Example 10: Diacetylspermine measurement 2)

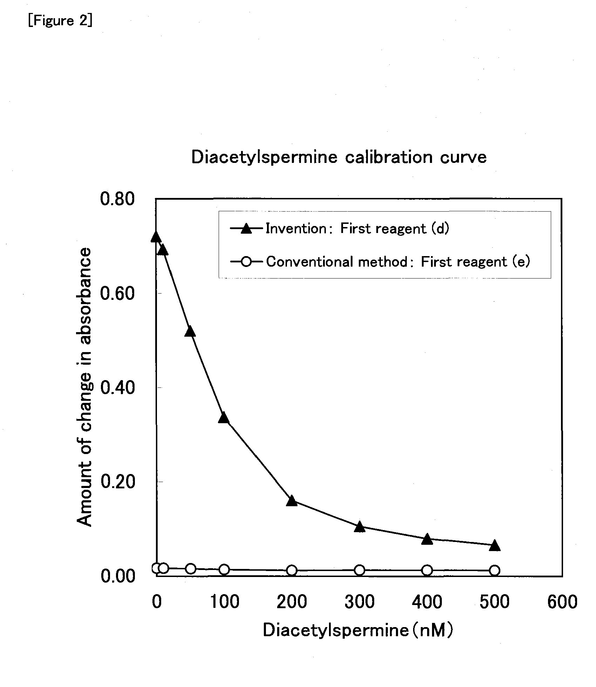

In this example, the two types of first reagents (d) and (e) for diacetylspermine measurement 2 as prepared in Example 9 above were used as the first reagent, and the diacetylspermine-bound colloidal gold reagent as prepared in Example 2 above was used as the second reagent. Diacetylspermine was dissolved in Bis-Tris (pH 7.4) containing 3.0 w/v% of BSA and 1.0 w/v% of sodium chloride so as to attain 0, 50, 100, 200, 300, 400, and 500 nM, giving diacetylspermine-containing samples. Then, 160 µL of the first reagent was added to 10 µL each of the diacetylspermine-containing samples and they were heated at 37°C for about 5 minutes, and 80 µL of the second reagent was added, and they were at 37°C for reaction. The amount of change in absorbance was measured with Hitachi 7070 automatic analyzer at photometric points from 18 to 31 at wavelengths of 546 nm and 660 nm. Figure 2 and Table 2 show the relationship between the diacetylspermine concentration and the amount of change in absorbance.

**[Table 2]**

| Diacetylspermine n(M) | First reagent | |
|---|---|---|
| | (d) | (e) |
| 0 | 0.7204 | 0.0169 |
| 10 | 0.6930 | 0.0170 |
| 50 | 0.5203 | 0.0161 |
| 100 | 0.3364 | 0.0136 |
| 200 | 0.1600 | 0.0123 |
| 300 | 0.1052 | 0.0128 |
| 400 | 0.0792 | 0.0129 |
| 500 | 0.0656 | 0.0121 |

As shown in Fig. 2 and Table 2, using the first reagent (e) which contained an anti-diacetylspermine antibody in the form of a single molecule, the occurrence of agglutination reaction was barely observed, and the amount of change in absorbance due to an agglutination reaction did not change in a manner dependent on the concentration of diacetylspermine, and it was thus not possible to measure the concentration. In contrast, using the first reagent (d) which contained a complex of anti-diacetylspermine antibody and avidin in the same amount of antibody as the amount of antibody in the first reagent (e), the occurrence of agglutination reaction was observed and the amount of change in absorbance due to the agglutination reaction was changed in a manner dependent on the concentration of the analyte diacetylspermine. Hence, the antibody could be used in the form of a complex to facilitate an agglutination reaction and to effect the inhibition of the agglutination reaction in a manner dependent of the concentration of the analyte (if any). Accordingly it was found that a complex having a plurality of molecules of the antibody bound therein allows for highly sensitive measurement also in a lower concentration range, thereby enabling measurement in a broader concentration range.

### Industrial Applicability

In the present invention, the agglutination reaction can be dramatically enhanced using a substance (for example, antibody) that specifically binds to an analyte in the form of a complex including a plurality of molecules of the substance that specifically binds to the analyte, rather than in the form of a single molecule, with latex particles or colloidal gold particles each having a plurality of molecules of an analyte or an analog of the analyte bound, and this results in significantly increased sensitivity to the measurement of the analyte. Therefore, according to the present invention, the measurement in such a low concentration range that could not be made with any conventional method becomes possible.

The method of the present invention does not require any agglutination enhancers, and is excellent in handling and reproducibility The method does not also require B/F separation and is highly suitable also for automation and can be applied to, for example, automatic analyzers broadly used in the field of clinical researches. Therefore, the method of the present invention is suitable as a method for immunological measurement of a trace component using an antigen-antibody reaction in the fields of industry, environment, and clinical research.

## Claims

1. A method for measuring an analyte in a sample, the method comprising:
(a) mixing:
a sample containing the analyte,
a complex comprising a plurality of molecules of a substance that specifically binds to the analyte, and
microparticles each having the analyte or an analog of the analyte bound to an insoluble support; and
(b) measuring an agglutination reaction of the microparticles in a mixture obtained in the step (a).

2. The method according to claim 1, wherein the complex is a substance having a plurality of molecules of an antibody against the analyte bound therein.

3. The method according to claim 1 or 2, wherein the analyte is a hapten and the analog of the analyte is a hapten-bound protein.

4. The method according to any one of claims 1 to 3, wherein the analog of the analyte is (i) a substance having a site of the analyte, (ii) a structural analog of the substance having the site, or (iii) a substance having a plurality of molecules of the substance having the site or the structural analog bound therein, which can be recognized by and bound to the substance that specifically binds to the analyte.

5. The method according to any one of claims 1 to 4, wherein the insoluble support is latex or colloidal gold.

6. A reagent kit for measurement comprising:
a first reagent, which comprises a complex comprising a plurality of molecules of a substance that specifically binds to an analyte; and
a second reagent, which comprises microparticles each having an analyte or an analog of the analyte bound to an insoluble support.

7. The kit according to claim 6, wherein the complex is a substance having a plurality of molecules of an antibody against the analyte bound therein.
